# EUROPEAN PATENT SPECIFICATION

(11) **EP 1 307 086 B1**
(45) Date of publication and mention of the grant of the patent: **14.11.2007**
(21) Application number: 01960771.2
(22) Date of filing: 06.07.2001
(51) Int. Cl.: A01G 7/00, A01G 23/00, A01H 4/00

(54) **PROCESS FOR PRODUCING CLONE SAPLINGS AND CUTTINGS**
VERFAHREN ZUR ERZEUGUNG VON KLONIERTEN BÄUMCHEN UND STECKLINGEN
PROCEDE DE PRODUCTION DE JEUNES ARBRES ET BOUTURES CLONES

(30) Priority: 07.07.2000 FI 20001628
(43) Date of publication of application: 07.05.2003
(73) Proprietor: M-real Oyj, 02100 Espoo (FI)
(72) Inventor: HERRALA, Kalervo, FIN-05200 Rajamäki (FI)
(74) Representative: Knuth-Lehtola, Sisko Hillevi
(86) International application number: PCT/FI2001/000648
(87) International publication number: WO 2002/003776

(56) References cited:
- PAUL REIM: 'Haava paljunemis-bioloogia, Die Vermehrungsbiologie der Aspe' TARTU ULIKOOLI METSAOSAKONNA TOIMETUSED, MITTEILUNGEN DER FORSTWISSENSCHAFTLICHEN ABTEILUNG DER UNIVERSITAT TARTU no. 16, 1930, pages XV - XVI, XP002948853
- DATABASE WPI Week 8507, Derwent Publications Ltd., London, GB; AN 1985-042572, XP002948854 & SU 1 079 215 A (MANGYSHLAG HORTIC) 15 March 1984
- GEORGE A. SCHIER: 'Vegetative propagation of Rocky Mountain aspen' USDA FOREST SERVICE GENERAL TECHNICAL REPORT, INTERMOUNTAIN FOREST AND RANGE EXPERIMENT STATION no. INT-44, 1978, pages 1 - 13, XP002948855
- R.B. HALL ET AL.: 'Commercial-scale vegetative propagation of aspens' GENERAL TECHNICAL REPORT - NORTH CENTRAL FOREST EXPERIMENT STATION, USDA FOREST SERVICE no. NC-140, 1990, pages 211 - 219, XP002948856
- ROBERT B. CAMPBELL: 'Asexual VS. sexual propagation of quaking aspen' GENERAL TECHNICAL REPORT, INTERMOUNTAIN FOREST AND RANGE EXPERIMENT STATION, USDA FOREST SERVICE no. INT-168, 1984, pages 61 - 65, XP002948857
- GEORGE A. SCHIER ET AL.: 'Effect of cold storage on development of suckers on aspen root cuttings' RESEARCH NOTE, INTERMOUNTAIN DOREST AND RANGE EXPERIMENT STATION, USDA FOREST SERVICE no. INT-248, 1978, pages 1 - 8, XP002948858
- DATABASE CABA [Online] DOC. NO. 760630973 DIMITROV KH. ET AL.: 'Methods of propagating populus alba', XP002948859 Retrieved from STN Database accession no. 76:39134 & GORSKOSTOPANSKA NAUKA vol. 12, no. 5, 1975, pages 34 - 42
- MARTTI LEPISTO: 'Metsan jalostus saatio, foundation for forest tree breeding' HAAVANJALOSTUKSEN TULOKSET KAYTTOON 1995, pages 5 - 7, XP002948860
- MARTTI LEPISTO: 'Metsan jalostus saatio, foundation for forest tree breeding' HYBRIDIHAAVAN MIKROTAIMITUOTANTOA KEHITETAAN 1996, page 23, XP002948861

## Description

The present invention relates to a method according to the preamble of claim 1 for producing clones and cuttings of woody plants.

Large numbers of high-quality cuttings are needed for forest planting. However, cutting production at the moment requires a lot of handwork, and mechanizing the processing phases of cuttings has not been successful to a considerable degree.

Lepistö's publication of (1995) describes a method, wherein the best aspen individuals are selected for cloning, and Lepistö's publication (1996) describes a method for growing clone cuttings from the selected aspen individuals by using the micro propagation method using, as the material, buds of a hybrid aspen in dormancy.

It has been previously known that aspen can be productively propagated from meristems and stem cuttings. However, the applicability of micro propagation from the meristems of aspen is restricted by high cutting production costs, which are mainly caused by the need for laboratory facilities and working. The applicability of stem cuttings is reduced by the low level of the propagation coefficient and rooting, which also causes high cutting production costs.

Reim's (1930) publication suggests a method for lifting the root system of the aspen from the ground and cutting it into root-cuttings, which are then planted in a greenhouse or a nursery. The Reim publication considers digging up and processing roots as difficult and expensive. Publication SU A 1079215 suggests the propagation of poplar, where the root system of the mother plant is cut off and planted to grow outside. The said publications have no mention of the selection and applicability of the mother plants to the production of originally clean clone cutting portions nor of the processing and selection of cuttings.

Aspen is used as a raw material in papermaking. However, great differences have been found between the properties of different aspen clones; therefore, from the point of view of the paper industry, it would be preferable, if the raw material could be obtained from certain, desired aspen clones. As, for example, the hybrid aspen reaches the size of saw-timber in as little as 25 to 30 years, growing aspen in comparison with other wood species would be a good alternative for the wood producer as well. However, the problem has been that there are no reliable and cost-effective methods for producing clones and cuttings from the desired aspen clones.

The purpose of the present invention is to eliminate the drawbacks related to prior art and to provide quite a new kind of solution for producing clones and cuttings of woody plants, particularly those of the tree species of the *Populus* genus or tree species that are reminiscent of the tree species of the *Populus* genus as to their rooting.

The invention is based on the discovery that root cuttings can be prepared from pieces that are mechanically isolated from the root system of the mother plant of the selected clone of the plant that is to be planted. The cuttings capable of rooting selected from them can be grown into plants and the plants can further be delivered to planters of wood, or the root cuttings can be planted in the ground directly.

The method according to the invention can be applied to different plant species, but it is especially well suited to producing clones of woody plants, which resemble the species of the *Populus* genus as to their rooting. The method is especially well suited to producing the clones of the species of the *Populus* genus, such as aspen (*Populus tremula*) and hybrid aspen, which is a cross between the Finnish and the North-American aspens (*Populus tremuloides*), or to producing the clones of other species of the *Populus* genus.

The invention provides considerable advantages. Thus, the method described herein can be used to produce large numbers of mutually identical plants from certain selected clones at very advantageous production costs. The method is as reliable as the micro propagation method, but the costs per plant are at least 20% lower. The cost savings are primarily due to the fact that cutting production can be transferred from laboratories to nurseries. The method is quick; cuttings can even be produced during one season of growth. Furthermore, the method according to the invention is easy to automate and mechanize.

According to the method, the clones and the mother plants from the clones are first selected in order to produce root cuttings ; the mother plants having been produced by using a suitable method. The mother plants can be produced, for example, by using a cloning method, such as micro propagation. The plant clones selected as the mother plants should have a genome that suits the purpose, and it must be possible to ensure the origin of the clones. The clones with a genome that suits the purpose refer to clones which, from the viewpoint of the end user, have the desired preferable properties, but also to clones in particular, which suit the method that is used. For the method used, especially those clones are suitable, which produce a great deal of root mass and which will differentiate well into a shoot and roots. Bare root or balled plants of 0.5-1 year of age are selected as mother plants. In the selection, attention is paid to the fact that the plants do not suffer from any damage caused by environmental factors, such as disease or pests. The mother plants are grown for at least one natural growing period or a part thereof to form a shoot and a root system. The natural growing period can be 0.5-1.5 years. The period can be shorter or longer than this. During the growing period, it is essential for the mother plant to form a good shoot and a strong and extensive root system.

When an open area is used for growing the mother plants, the area must be fallowed completely in order to ensure that the clones remain clear of other clones or closely related plants.

According to a preferred embodiment of the invention, the mother plants are arranged at a distance of 40-100 cm from each other. It is also preferable to arrange the mother plants in at least two adjacent rows, the distance between which is approximately about 100-500 cm, whereby a fertilizer line is arranged between the rows. To advance the growth of roots, a nitrogenous fertilizer can be used. This embodiment provides the advantage that the roots grow fairly straight, whereby it is easy to process them.

After the growing period, the cultivated mother plants are left in dormancy. Dormancy refers to a state, where the photosynthesis, breathing, and water consumption of the plant are at their minimum. In a greenhouse, this means that the plants are kept in minor light in a cool place, and not watered very often. In nature, this means that the vital functions of the plant are slowed down after the season of growth with the amount of light and the temperature decreasing. When going into dormancy, the nutrients of the plant accumulate in the root system and the plant better withstands various treatments, such as cutting the shoot, transferring the plant or shortening the roots. In this case, it is essential that the root system of the plant withstands the mechanical treatment it is subjected to.

After the mother plants have reached complete dormancy, the shoots of the plants are cut. If the shoots are not cut, the plant might use the nutrients accumulated in the root system for growing a shoot, thus weakening the ability of the root system to take root. Cutting is carried out at a height of 5-40 cm, preferably at a height of about 20 cm. The stub of about 20 cm that is left on the mother plant is a suitable place for gripping, when processing the root system. It is preferable to recover the root system of the mother plant at the beginning of the plant cultivation, which means that the root system is further processed at the beginning of the growing season right after unearthing the mother plant or bringing it from a greenhouse or a cold store. The thinnest part of the mother plants' root system is removed from the root system and roots with a diameter of more than 3 mm are cut to form root cuttings. Roots with even smaller diameter could also be included, but they are more difficult to process and their ability to form a new root system and shoots is limited. Furthermore, it is preferable to select as root cuttings the basal or intermediate part of the root. The length of the piece of root can be about 2-10 cm, preferably about 3-6 cm. It is most practical and quick to mechanically clean, sort, and cut the roots of the mother plants into pieces.

The pieces of root, which here are called root cuttings, are brought into rooting conditions to be rooted on a suitable growth medium. Such a suitable growth medium can be peat sand, for example. The growth medium can be, for example, in cells. A thin layer (such as 1 cm) of growth medium can be spread on top of the pieces of root.

It is especially preferable to make the root cuttings root in an essentially horizontal position. In practice, this means a position, where the longitudinal axis of the root cutting is at an angle less than 45 degrees in relation to the horizontal plane.

The root cuttings inserted into the growth medium are rooted in conditions, where the temperature and the moisture are kept suitable, such as a greenhouse. The lighting can consist of natural light, for example.

The root cuttings are kept in rooting conditions long enough to show signs of shoots and/or roots beginning to grow. At that time, the shoots have reached a size of 3-10 cm, for example, and the roots have begun or are beginning to grow. This period can be about 4-8 weeks in length; indeed, for certain clones or in certain growing conditions, this period can be as long as 12 weeks, which is affected by both the growing conditions, the clone, and the length and the thickness of the piece of root. After this, the root cuttings that have rooted or formed a shoot are transplanted, i.e., transferred with their root balls to grow in a new growth medium (e.g., an almost pure garden peat medium), where the shoots and the roots have room to grow and become rooted cuttings of the desired size (a larger root ball).

The reliability and the effectiveness of the method can further be increased by stratifying, i.e., pre-treating the root cuttings in conditions that correspond to the rooting conditions described above before inserting them into the growth medium to identify cuttings that root or are capable of rooting. With the aid of light, moisture and, optionally, hormones, such as cytokinin, the pre-treatment can be used to create conditions that contribute to sprouting in particular. In the pre-treatment, the pieces of root are preferably placed densely next to each other on a horizontal plane on top of a suitable medium, and they are not buried into the growth medium. They can be covered with light gauze or paper to maintain suitable humidity. In the root cuttings, the differentiation of the shoot begins from the proximal end of the piece of root and appears as a bulge on the surface of the root. The root cuttings are kept in such conditions for about 10 to 15 days, for example, whereby the skin of the root begins to develop lumps visible to the eye, which indicate that the shoot and/or the root have begun to grow. On the basis of these signs, the root cuttings that root well can be distinguished from those that root slowly or do not root at all. In the conditions described above, sprouting takes place within a few weeks, during which time the root cuttings that have started to produce shoots are pricked out for further growing. In the transplanting phase, the root cuttings to be pricked out can be treated with hormones, such as auxins, to advance root formation.

After pricking out, the so-called implanting of the rooted cuttings developed from the root cuttings can be carried out in plastic houses. Normally, for the cuttings of aspen pricked out from rooting cells, for example, this takes a few days (3 to 6 days) only, for the cuttings pricked out from those stratified, this time is longer (6 to 10 days). In that case, the pricked out plant takes root in the new growth medium and withstands a transfer to open land, among others, where the actual growing takes place. An outdoor growing field requires good irrigators and effective mechanical devices for solute fertilization and pest control.

The root cuttings can also be inserted directly into the growing medium used for pricking out, which is kept in rooting conditions for 3 to 8 weeks or longer, after which the rooted root cuttings are transferred to outdoor growing. In the pricking out, normally, fertilized garden peat suitable for the plant in question is used as a growth medium. The time required depends on whether stratified or unstratified root cuttings are use. This process requires a larger space in the plastic house than the pricking out process mentioned above.

Any plants grown from the root cuttings are unearthed after they have wintered. Wintering here means that the plants have reached dormancy, i.e., growing has stopped and, for example, buds have been formed and the leaves have turned yellow and fallen. When classifying the plants of the clone aspen intended for forest cultivation, for example, the classification standards for birch plants can be used, which are defined by the Commercial Code 684/79 on Forest Reproductive Material and by the Resolutions 271/91 and 1210/94 of the Ministry of Agriculture and Forestry on the Marketing of Forest Reproductive Material. The inventory of the plants is carried out per clones, whereby it is, of course, preferable to mark the packages.

It should be noted that in all the handling and growing phases of the method, the clones should be kept apart from each other until the end of the process.

The plants can be stored for winter either in a cold store or the plant bags can be kept in winter storage outdoors.

For autumn planting of forests, the plants can be delivered towards the end of the growing period, at the end of the summer (in Finland, around the middle of August). At that time, caution should be exercised in transporting and handling the plants in order not to damage the plants.

For spring planting, the plant bags must be melted before transportation and planting. If the plants have been stored in cold storage, the plants should be immersed in water for about 1 day before planting for giving them a fresh supply of water.

The planted area should be suitable for the purpose and the soil should be prepared. The number of clones should be in accordance with the regulations. In connection with planting, protection against moles and hares should be set; in areas exposed to deer and elk damage, in turn, electric or wire-net fences should be built.

Growing root cuttings into plants and delivering the plants to the areas of cultivation are described above. Another possibility is to directly insert the root cuttings into selected, good areas under cultivation, such as land with good mull and warm soil. In that case, the phase of growing plants is omitted. The root cuttings obtained from cutting the roots, selecting the rooted cuttings or pre-treatment, i.e., stratification, are selected and packed in delivery boxes developed for the purpose, wherein the disadvantages of heat, moisture, and vibration have been minimized, and the cuttings are sent directly to the area under cultivation.

When root cuttings are transported to areas of cultivation, the transportation requires an accurate delivery plan between the buyer and the seller. The root cuttings can be delivered by parcel post to an area of as large as 2 hectares, whereby the weight is less than 20 kg. The time used for transportation should not exceed 1 day.

The roots are planted in or "inserted" into the area under cultivation, which has been prepared; the soil preferably has good moisture properties, contains 3-6% organic matter, and is warm. The most preferable time to insert the cuttings into the ground would be at the beginning of the growing period, between the end of spring and early summer (in Finland, from the middle of May to the middle of June; from 20 May to 10 June), when the ground is suitably warm but not dried. It is preferable to mix a planting medium from the foundation soil of the cultivation area at the point of planting the cutting. The root cutting should be set into the medium exactly according to the instructions, and a plant screen should be placed on top of the cutting.

Later on, after about 1 to 1.5 months, (in Finland, at the beginning of July), it can be checked, how the cuttings have started to grow and assessed, whether further planting is needed. Further planting is preferably carried out with cuttings after a couple of months from the planting (in Finland, at the turn of July and August).

The method according to the invention can be used to produce the clones of the wood species of the *Populus* genus in particular. These can be, for example, the clones of *P*. *tremula, P. tremuloides, P balsamea, P. balsamifera, P. trichocarpa, P. heterophylla, P. deltoides, P. grandidentata, an aspen species, such as P. tremula x tremuloides, P. tremula x tremula, P. deltoides x trichocarpa, P. trichocarpa x deltoides, P. deltoides x nigra, P. maximowiczii x trichocarpa,* which is hybridized from stock aspens, or the clone s of another species produced by gene technology, or the clones of poplar.

The method according to the invention can also be used to produce the clones of other woody plants, which have similar rooting properties as the species of the *Populus* genus (e.g., plum or cherry with cultivated roots). The method can be used to produce, for example, the clones of various park bushes or trees or the clones of raspberry.

The following non-limiting examples describe the invention in detail:

### Example 1

In the test, different alternatives to arrange the roots of hybrid aspen were compared. The percentage of rooting (the portion of the root cuttings, which had formed a shoot and a root, of all the root cuttings) refers to the rooting result at the end of June (by 21-22 June), the total rooting % refers to the rooting result about one month later (by 19 July), when the planting of the root cuttings had taken place in the middle of May (11 to 18 May). The roots that were arranged horizontally definitely gave the best rooting result, as seen in Table 1.

**Table 1**

| **Positioning** | **Positioning test** | |
|---|---|---|
| | **Rooting %** | **Total rooting %** |
| Vertically | 24.0 % | 41.3 % |
| Upside down | 7.3 % | 26.0 % |
| Horizontally | 73.6 % | 84.0 % |
| All positions | 35.0 % | 50.5 % |

### Example 2

The test compared the of various alternative fertilizers on the rooting results of the root cuttings of hybrid aspen, given to the mother plants during the growing period. Kekkilä Super X 5 and Super X 7 are both multi-nutrient fertilizers, but Kekkilä Super X 7 contains no nitrogen. The rooting % and the total rooting % were defined as in Example 1.

**Table 2.**

| **Fertilization** | **Actual test** | | **Mass test** | | **Whole test** | |
|---|---|---|---|---|---|---|
| | **Rooting %** | **Total rooting %** | **Rooting %** | **Total rooting %** | **Rooting %** | **Total rooting %** |
| Not fertilized | 40.5 % | 55.5 % | 31.6 % | 46.2 % | 34.9 % | 48.8 % |
| Kekkilä SuperX 5 | 39.7 % | 58.3 % | 37.1 % | 51.4 % | 38.1 % | 54.1 % |
| Kekkilä SuperX 7 | 35.9 % | 55.8 % | 32.7 % | 47.6 % | 34.5 % | 51.5 % |
| All fertilizations | 38.3 % | 56.9 % | 34.6 % | 49.1 % | 36.2 % | 52.3 % |

The results in Table 2 indicate that fertilization with the nitrogenous fertilizer in particular improves the total rooting result.

### Example 3

When comparing the ability of the different parts of the hybrid aspen roots to take root, it was discovered that the basal and intermediate parts of the roots were the most effective parts to take root, as Table 3 indicates. The rooting and total rooting % were defined as in Example 1.

**Table 3**

| **Part of root** | **Actual test** | |
|---|---|---|
| | **Rooting %** | **Total rooting %** |
| Base | 54.7 % | 68.4 % |
| Intermediate part | 31.8 % | 52.8 % |
| Tip | 18.8 % | 36.3 % |
| All parts of root | 37.2 % | 54.6 % |

### Example 4

When examining the effect of the time of cutting the shoots of unfertilised hybrid aspen mother plants on the rooting property, it was discovered that cutting obviously had an advantageous effect on taking root. The rooting and total rooting % were defined as in Example 1. As Table 4 shows, the best result was obtained, when the cutting of shoots was performed on plants in complete dormancy (10 December).

**Table 4**

| **Cutting** | **Actual test** | | **Mass test** | | **Whole test** | |
|---|---|---|---|---|---|---|
| | **Rooting %** | **Total rooting %** | **Rooting %** | **Total rooting %** | **Rooting %** | **Total rooting %** |
| No cutting | 29.0 % | 41.0 % | 18.1 % | 24.2 % | 22.2 % | 32.2 % |
| 24.9.1998 | 27.0 % | 40.5 % | 19.6 % | 31.1 % | 23.0 % | 34.3 % |
| 10.12.1998 | 49.0 % | 67.0 % | 40.6 % | 56.6 % | 42.6 % | 59.3 % |
| 23.4.1999 | 40.5 % | 55.5 % | 31.6 % | 46.2 % | 34.9 % | 48,8 % |
| All cuttings | 36.4 % | 51.0 % | 28.1 % | 40.6 % | 31.0 % | 44.1 % |

## Claims

1. A method for producing clones of woody plants, **characterized in that**
- the mother plants are selected from selected clones in order to produce root cuttings
- the mother plants are grown for at least one natural growing period or part thereof for forming a shoot and a root system,
- the grown mother plants are left in dormancy after the growing period,
- the shoots of the mother plants are cut after the plants have reached complete dormancy,
- the root system of the mother plant is recovered,
- the thinnest portion of the mother plant root system is removed and the roots of a diameter larger than 3 mm are cut into root cuttings, which are optionally stratified,
- the root cuttings are brought to rooting conditions to take root in a suitable growth medium,
- the root cuttings, which have started to grow a shoot within eight weeks from planting and taken root, or which show signs of starting to grow shoots and/or a root, are selected and recovered, and
- the selected root cuttings are grown to become plants

2. A method according to claim 1, **characterized in that** the shoots of the mother plants are cut at a height of 5 to 40 cm, preferably about 20 cm.

3. A method according to claim 1 or 2, **characterized in that** the root system of the mother plants is recovered at the beginning of the plant cultivation.

4. A method according to any one of claims 1 to 3, **characterized in that** the mother plants are produced by the micro propagation method or some other reliable cloning method.

5. A method according to any one of claims 1 to 4, **characterized in that** plants with bare roots or ball plants of 0.5 to 1 year of age are selected as mother plants.

6. A method according to any one of the preceding claims, **characterized in that** the growing period of the mother plants is 0.5-1.5 years.

7. A method according to any one of the preceding claims, **characterized in that**, for cultivation, the mother plants are arranged at a distance of 40 to 100 cm from each other.

8. A method according to any one of the preceding claims, **characterized in that** the mother plants are arranged in at least two adjacent rows, the distance between them being approximately about 100 to 500 cm, whereby a fertilizing line is arranged between the rows.

9. A method according to any one of the preceding claims, **characterized in that**, in root production, a nitrogenous fertilizer is used to advance the growth of the roots.

10. A method according to any one of the preceding claims, **characterized in that** the roots of the mother plants, which are less than 3 mm in thickness, are cut off and the roots with a diameter larger than this are cut into pieces.

11. A method according to claim 10, **characterized in that** the basal or intermediate part of the root is selected as a root cutting.

12. A method according to any one of the preceding claims, **characterized in that** a piece of root with a length of about 2 to 10 cm, preferably about 3 to 6 cm, is selected as a root cutting.

13. A method according to any one of the preceding claims, **characterized in that** the root cuttings are placed on a peat sand base to take root.

14. A method according to any one of the preceding claims, **characterized in that** the root cuttings are positioned in an essentially horizontal position to take root.

15. A method according to any one of the preceding claims, **characterized in that** the root cuttings are stratified before inserting them into the growth medium.

16. A method according to any one of the preceding claims, **characterized in that** the root cuttings are kept in rooting conditions long enough for them to show signs of starting to grow shoots and/or roots.

17. A method according to any one of the preceding claims, **characterized in that** the selected root cuttings are pricked out and grown to become plants.

18. A method according to any one of the preceding claims, **characterized in that** the plants developed from the root cuttings are unearthed, when they have become ready for the winter.

19. A method according to any one of claims 1 to 16, **characterized in that** the selected root cuttings are inserted directly into the selected area of cultivation.

20. A method according to claim 19, **characterized in that** a planting medium is mixed from the basic soil of the cultivation area at the planting point of the cutting.

21. A method according to any one of the preceding claims, **characterized in that** the clones of the tree species of the *Populus* genus are produced.

22. A method according to claim 21, **characterized in that** the clones of the *P*. *tremula, P. tremuloides, P balsamea, P. balsamifera, P. trichocarpa, P. heterophylla, P. deltoides, P. grandidentata, an aspen species hybridised from stock aspens, such as P. tremula x tremuloides, P. tremula x tremula, P. deltoides x trichocarpa, P. trichocarpa x deltoides, P. deltoides x nigra, P. maximowiczii x trichocarpa* or another species produced by gene technology, or the clones of poplar are produced.

23. A method according to any one of claims 1 to 20, **characterized in that** the clones of park bushes or trees or those of raspberry are produced.

24. A method for producing the clones of woody plants, **characterized in that**
- the mother plants are selected from selected clones in order to produce root cuttings;
- the mother plants are grown for at least one natural growing period or part thereof for forming a shoot and a root system,
- the grown mother plants are left in dormancy after the growing period,
- the shoots of the mother plants are cut after the plants have reached complete dormancy,
- the root system of the mother plant is recovered,
- the thinnest portion of the mother plant root system is removed and the roots of a diameter larger than 3 mm are cut into root cuttings, which are optionally stratified,
- the root cuttings are placed in rooting conditions to take root in a suitable growth medium,
- the root cuttings, which have started to grow a shoot within eight weeks from planting and taken root, or which show signs of starting to grow shoots and/or a root, are selected and recovered, and
- the selected root cuttings or the stratified root cuttings are inserted directly into the selected area of cultivation.

25. A method according to claim 24, **characterized in that** a planting medium is mixed from the base soil of the cultivation area at the planting point of the cutting.

## Patentansprüche

1. Verfahren zum Produzieren von Clonen holziger Pflanzen, **dadurch gekennzeichnet, dass**
- die Mutterpflanzen aus ausgewählten Clonen ausgewählt werden, um Wurzelschnitte herzustellen,
- die Mutterpflanzen für mindestens eine natürliche Wachstumsperiode oder einen Teil davon gezüchtet werden, um einen Schössling und ein Wurzelsystem zu bilden,
- die gewachsenen Mutterpflanzen nach der Wachstumsperiode im Ruhezustand gelassen werden,
- die Schösslinge der Mutterpflanzen geschnitten werden, nachdem die Pflanzen den vollständigen Ruhezustand erreicht haben,
- das Wurzelsystem der Mutterpflanzen gewonnen wird,
- der dünnste Teil des Wurzelsystems der Mutterpflanze entfernt wird und die Wurzeln mit einem Durchmesser größer als 3 mm in Wurzelschnitte geschnitten werden, die gegebenenfalls geschichtet werden,
- die Wurzelschnitte in Wurzelbedingungen gebracht werden, um in einem geeigneten Wachstumsmedium zu wurzeln,
- die Wurzelschnitte, die innerhalb von acht Wochen nach Pflanzung begonnen haben, einen Schössling auszubilden und die gewurzelt haben oder die Anzeichen zeigen, dass sie beginnen, Schösslinge und/oder eine Wurzel auszubilden, ausgewählt und gewonnen werden, und
- die ausgewählten Wurzelschnitte gezüchtet werden, um Pflanzen zu werden.

2. Verfahren nach Anspruch 1, **dadurch gekennzeichnet, dass** die Schösslinge der Mutterpflanze bei einer Höhe von 5 bis 40 cm, vorzugsweise etwa 20 cm, geschnitten werden.

3. Verfahren nach Anspruch 1 oder 2, **dadurch gekennzeichnet, dass** das Wurzelsystem der Mutterpflanzen zu Beginn des Pflanzenanbaus gewonnen wird.

4. Verfahren nach einem der Ansprüche 1 bis 3, **dadurch gekennzeichnet, dass** die Mutterpflanzen durch das Mikropropagationsverfahren oder durch andere verlässliche Clonierungsverfahren hergestellt werden.

5. Verfahren nach einem der Ansprüche 1 bis 4, **dadurch gekennzeichnet, dass** Pflanzen mit nackten Wurzeln oder Ballenpflanzen mit einem Alter von 0,5 bis 1 Jahr als Mutterpflanzen ausgewählt werden.

6. Verfahren nach einem der vorhergehenden Ansprüche, **dadurch gekennzeichnet, dass** die Wachstumsperiode der Mutterpflanzen 0,5 bis 1,5 Jahre ist.

7. Verfahren nach einem der vorhergehenden Ansprüche, **dadurch gekennzeichnet, dass** die Mutterpflanzen für die Kultivierung in einem Abstand von 40 bis 100 cm voneinander angeordnet werden.

8. Verfahren nach einem der vorhergehenden Ansprüche, **dadurch gekennzeichnet, dass** die Mutterpflanzen in mindestens zwei benachbarten Reihen angeordnet werden, wobei der Abstand zwischen ihnen ungefähr etwa 100 bis 500 cm beträgt, wobei eine Düngelinie zwischen den Reihen angeordnet ist.

9. Verfahren nach einem der vorhergehenden Ansprüche, **dadurch gekennzeichnet, dass** für die Wurzelherstellung ein Stickstoffdünger verwendet wird, um das Wachstum der Wurzeln zu fördern.

10. Verfahren nach einem der vorhergehenden Ansprüche, **dadurch gekennzeichnet, dass** die Wurzeln der Mutterpflanzen, die weniger als 3 mm dick sind, abgeschnitten werden und die Wurzeln mit einem größeren Durchmesser in Stücke geschnitten werden.

11. Verfahren nach Anspruch 10, **dadurch gekennzeichnet, dass** der basale oder der mittlere Teil der Wurzel als Wurzelschnitt ausgewählt wird.

12. Verfahren nach einem der vorhergehenden Ansprüche, **dadurch gekennzeichnet, dass** ein Stück Wurzel mit einer Länge von etwa 2 bis 10 cm, vorzugsweise etwa 3 bis 6 cm, als Wurzelschnitt ausgewählt wird.

13. Verfahren nach einem der vorhergehenden Ansprüche, **dadurch gekennzeichnet, dass** die Wurzelschnitte auf eine Torf-Sand-Basis gesetzt werden, um zu wurzeln.

14. Verfahren nach einem der vorhergehenden Ansprüche, **dadurch gekennzeichnet, dass** die Wurzelschnitte in einer im Wesentlichen horizontalen Position platziert werden, um zu wurzeln.

15. Verfahren nach einem der vorhergehenden Ansprüche, **dadurch gekennzeichnet, dass** die Wurzelschnitte geschichtet werden, bevor sie in das Wachstumsmedium eingebracht werden.

16. Verfahren nach einem der vorhergehenden Ansprüche, **dadurch gekennzeichnet, dass** die Wurzelschnitte in Wurzelbedingungen gehalten werden, die lang genug sind, dass sie Zeichen beginnenden Wachstums von Schösslingen und/oder Wurzeln zeigen.

17. Verfahren nach einem der vorhergehenden Ansprüche, **dadurch gekennzeichnet, dass** die ausgewählten Wurzelschnitte ausgestochen und gezüchtet werden, um Pflanzen zu werden.

18. Verfahren nach einem der vorhergehenden Ansprüche, **dadurch gekennzeichnet, dass** die Pflanzen, die sich aus den Wurzelschnitten entwickeln, ausgegraben werden, sobald sie bereit sind für den Winter.

19. Verfahren nach einem der Ansprüche 1 bis 16, **dadurch gekennzeichnet, dass** die ausgewählten Wurzelschnitte direkt in das ausgewählte Kultivierungsgebiet eingebracht werden.

20. Verfahren nach Anspruch 19, **dadurch gekennzeichnet, dass** zum Zeitpunkt der Einpflanzung des Schnittes ein Pflanzenmedium aus der basischen Erde des Kultivierungsgebiets gemischt wird.

21. Verfahren nach einem der vorhergehenden Ansprüche, **dadurch gekennzeichnet, dass** die Clone der Baumarten der Gattung *Populus* hergestellt werden.

22. Verfahren nach Anspruch 21, **dadurch gekennzeichnet, dass** die Clone von *P*. *tremula, P. tremuloides, P. balsamea, P. balsamifera, P. trichocarpa, P. heterophylla, P. deltoides, P. grandidentata,* eine Espenart hybridisiert aus Stammespen, wie z. B. *P*. *tremula x tremuloides, P. tremula x tremula, P. deltoides x trichocarpa, P. trichocarpa x deltoides, P. deltoides x nigra, P. maximowiczii x trichocarpa* oder einer anderen Art, die durch Gentechnologie produziert wurde, oder die Clone von Pappeln hergestellt werden.

23. Verfahren nach einem der Ansprüche 1 bis 20, **dadurch gekennzeichnet, dass** die Clone von Parkbüschen oder Bäumen oder diejenigen von Himbeeren hergestellt werden.

24. Verfahren zur Herstellung der Clone holziger Pflanzen, **dadurch gekennzeichnet, dass**
- die Mutterpflanzen aus ausgewählten Clonen ausgewählt werden, um Wurzelschnitte herzustellen,
- die Mutterpflanzen für mindestens eine natürliche Wachstumsperiode oder einen Teil davon gezüchtet werden, um einen Schössling und ein Wurzelsystem zu bilden,
- die gewachsenen Mutterpflanzen nach der Wachstumsperiode im Ruhezustand gelassen werden,
- die Schösslinge der Mutterpflanzen geschnitten werden, nachdem die Pflanzen den vollständigen Ruhezustand erreicht haben,
- das Wurzelsystem der Mutterpflanzen gewonnen wird,
- der dünnste Teil des Wurzelsystems der Mutterpflanze entfernt wird und die Wurzeln mit einem Durchmesser größer als 3 mm in Wurzelschnitte geschnitten werden, die gegebenenfalls geschichtet werden,
- die Wurzelschnitte in Wurzelbedingungen gebracht werden, um in einem geeigneten Wachstumsmedium zu wurzeln,
- die Wurzelschnitte, die innerhalb von acht Wochen nach Pflanzung begonnen haben, einen Schössling auszubilden und die gewurzelt haben oder die Anzeichen zeigen, dass sie beginnen, Schösslinge und/oder eine Wurzel auszubilden, ausgewählt und gewonnen werden, und
- die ausgewählten Wurzelschnitte oder die geschichteten Wurzelschnitte direkt in das ausgewählte Kultivierungsgebiet eingefügt werden.

25. Verfahren nach Anspruch 24, **dadurch gekennzeichnet, dass** zum Zeitpunkt der Einpflanzung des Schnittes ein Pflanzenmedium aus der basischen Erde des Kultivierungsgebiets gemischt wird.

## Revendications

1. Procédé de production de clones de plantes ligneuses, **caractérisé en ce que** :
- les plantes mères sont choisies à partir de clones choisis afin de produire des boutures de racine,
- les plantes mères effectuent leur croissance pendant au moins une période de croissance naturelle ou une partie de celle-ci pour former un système foliacé et un système radiculaire,
- les plantes mères qui ont effectué leur croissance sont laissées en dormance pendant la période de croissance,
- les pousses des plantes mères sont coupées après que les plantes ont atteint une dormance complète,
- le système radiculaire de la plante mère est récupéré,
- la partie la plus fine du système radiculaire de la plante mère est retirée et les racines d'un diamètre supérieur à 3 mm sont coupées en des boutures de racine, qui sont éventuellement stratifiées,
- les boutures de racine sont amenées à des conditions d'enracinement pour qu'elles prennent racine dans un milieu de croissance approprié,
- les boutures de racine, qui ont commencé à faire croître une pousse en huit semaines à partir de la plantation et qui ont pris racine, ou qui montrent des signes de début de croissance de pousse et/ou d'une racine, sont choisies et récupérées, et
- les boutures de racine choisies effectuent leur croissance pour devenir des plantes.

2. Procédé selon la revendication 1, **caractérisé en ce que** les pousses des plantes mères sont coupées à une hauteur de 5 à à 40 cm, de préférence d'environ 20 cm.

3. Procédé selon la revendication 1 ou 2, **caractérisé en ce que** le système radiculaire des plantes mères est récupéré au début de la culture de plante.

4. Procédé selon l'une quelconque des revendications 1 à 3, **caractérisé en ce que** les plantes mères sont produites par le procédé de micro propagation ou un certain autre procédé de clonage fiable.

5. Procédé selon l'une quelconque des revendications 1 à 4, **caractérisé en ce que** les plantes à racine nue ou les plantes en motte âgées de 0,5 à 1 an sont choisies en tant que plantes mères.

6. Procédé selon l'une quelconque des revendications précédentes, **caractérisé en ce que** la période de croissance des plantes mères est de 0,5 à 1,5 an.

7. Procédé selon l'une quelconque des revendications précédentes, **caractérisé en ce que**, pour une culture, les plantes mères sont agencées à une distance de 40 à 100 cm les unes des autres.

8. Procédé selon l'une quelconque des revendications précédentes, **caractérisé en ce que** les plantes mères sont agencées en au moins deux rangées adjacentes, la distance entre elles étant approximativement d'environ 100 à 500 cm, de sorte qu'une ligne de fertilisation soit agencée entre les rangées.

9. Procédé selon l'une quelconque des revendications précédentes, **caractérisé en ce que**, dans une production de racine, un engrais azoté est utilisé pour faire progresser la croissance des racines.

10. Procédé selon l'une quelconque des revendications précédentes, **caractérisé en ce que** les racines des plantes mères, qui sont épaisses de moins de 3 mm, sont coupées et **en ce que** les racines ayant un diamètre supérieur à celui-ci sont coupées en morceaux.

11. Procédé selon la revendication 10, **caractérisé en ce que** la partie basale ou intermédiaire de la racine est choisie en tant que bouture de racine.

12. Procédé selon l'une quelconque des revendications précédentes, **caractérisé en ce qu'**un morceau de racine ayant une longueur d'environ 2 à 10 cm, de préférence d'environ 3 à 6 cm, est choisi en tant que bouture de racine.

13. Procédé selon l'une quelconque des revendications précédentes, **caractérisé en ce que** les boutures de racine sont placées sur une base de tourbe et de sable pour qu'elles prennent racine.

14. Procédé selon l'une quelconque des revendications précédentes, **caractérisé en ce que** les boutures de racine sont positionnées en une position essentiellement horizontale pour prendre racine.

15. Procédé selon l'une quelconque des revendications précédentes, **caractérisé en ce que** les boutures de racine sont stratifiées avant insertion de celles-ci dans le milieu de croissance.

16. Procédé selon l'une quelconque des revendications précédentes, **caractérisé en ce que** les boutures de racine sont gardées en des conditions d'enracinement suffisamment longtemps pour qu'elles montrent des signes de début de croissance de pousses et/ou de racines.

17. Procédé selon l'une quelconque des revendications précédentes, **caractérisé en ce que** les boutures de racine choisies sont repiquées et effectuent leur croissance pour devenir des plantes.

18. Procédé selon l'une quelconque des revendications précédentes, **caractérisé en ce que** les plantes développées à partir des boutures de racine sont déterrées, quand elles sont devenues prêtes pour l'hiver.

19. Procédé selon l'une quelconque des revendications 1 à 16, **caractérisé en ce que** les boutures de racine choisies sont insérées directement dans la zone choisie de culture.

20. Procédé selon la revendication 19, **caractérisé en ce qu'**un milieu de plantation est mélangé à partir du sol basique de la zone de culture au point de plantation de la bouture.

21. Procédé selon l'une quelconque des revendications précédentes, **caractérisé en ce que** les clones de l'espèce d'arbre du genre *Populus* sont produits.

22. Procédé selon la revendication 21, **caractérisé en ce que** les clones de *P. tremula, P. tremuloides, P. balsamea, P*. *balsamifera, P. trichocarpa, P. heterophylla, P. deltoides, P. grandidentata,* une espèce de tremble hybridé à partir de trembles mères, telle que *P. tremula x tremuloides, P. tremula x tremula, P. deltoides x trichocarpa, P. trichocarpa x deltoides, P. deltoides x nigra, P. maximowiczzi x trichocarpa* ou une autre espèce produite par technologie génique, ou les clones de peupliers sont produits.

23. Procédé selon l'une quelconque des revendications 1 à 20, **caractérisé en ce que** les clones d'arbustes ou d'arbres de parc ou ceux de framboisier sont produits.

24. Procédé de production de clones de plantes ligneuses, **caractérisé en ce que**
- les plantes mères sont choisies à partir de clones choisis afin de produire des boutures de racine,
- les plantes mères effectuent leur croissance pendant au moins une période de croissance naturelle ou une partie de celle-ci pour former un système foliacé et un système radiculaire,
- les plantes mères effectuant leur croissance sont laissées en dormance pendant la période de croissance,
- les pousses des plantes mères sont coupées après que les plantes ont atteint une dormance complète,
- le système radiculaire de la plante mère est récupéré,
- la partie la plus fine du système radiculaire de la plante mère est retirée et les racines d'un diamètre supérieur à 3 mm sont coupées en des boutures de racine, qui sont éventuellement stratifiées,
- les boutures de racine sont amenées à des conditions d'enracinement pour qu'elles prennent racine dans un milieu de croissance approprié,
- les boutures de racine, qui ont commencé à faire croître une pousse en huit semaines à partir de la plantation et qui ont pris racine, ou qui montrent des signes de début de croissance de pousse et/ou d'une racine, sont choisies et récupérées, et
- les boutures de racine choisies ou les boutures de racine stratifiées sont insérées directement dans la zone choisie de culture.

25. Procédé selon la revendication 24, **caractérisé en ce qu'**un milieu de plantation est mélangé à partir du sol de base la zone de culture au point de plantation de la bouture.
